# EUROPÄISCHE PATENTANMELDUNG

(11) **EP 3 574 873 A1**
(43) Veröffentlichungstag der Anmeldung: **04.12.2019**
(21) Anmeldenummer: 18174805.4
(22) Anmeldetag: 29.05.2018
(51) Int. Cl.: A61F 2/966, A61F 2/915

(54) **KATHETERSYSTEM ZUM IMPLANTIEREN EINES SELBSTEXPANDIERENDEN STENTS**

(71) Anmelder: BIOTRONIK AG, 8180 Bülach (CH)
(72) Erfinder: Fargahi, Amir, 8180 Bülach (CH)
(74) Vertreter: Randoll, Sören

(57) **Zusammenfassung**

Die Erfindung betrifft ein Kathetersystem (1) zum Implantieren eines selbstexpandieren den Stents (2), wobei erfindungsgemäß zwischen dem Stent (2) und einem Innenschaft (40) des Kathetersystems (40) ein separates Abstützelement (60) vorgesehen ist, das den Innenschaft (40) umgibt und das dazu ausgebildet ist, den Stent (2) beim Bewegen eines den Stent (2) umgebenden Außenschaftes (30) des Kathetersystems (1) aus einer ersten Position in eine zweite Position abzustützen, wenn ein am Innenschaft (40) festgelegter Stentstopper (50) zum Freisetzen des Stents (2) in der axialen Richtung (x) gegen das proximale Ende (2a) des Stents (2) drückt.

## Beschreibung

Die Erfindung betrifft ein Kathetersystem zum Implantieren eines selbstexpandierenden Stents.

Bei derartigen Kathetersystemen sind aus dem Stand der Technik Freisetzungsmechanismen für den Stent bekannt, bei denen ein Stentstopper auf einem Innenschaft des Katheters fixiert ist, wobei der gecrimpte Stent auf einer Innenseite eines den Innenschaft umgebenden Außenschaftes des Katheters sitzt. Beim Zurückbewegen des Außenschaftes gegenüber dem Innenschaft drückt der Stentstopper gegen ein proximales Ende des Stents, so dass der Stent beim Zurückziehen des Außenschaftes freigesetzt wird, wobei er sich durch Selbstexpansion aufstellt. Eine solche Kathetereinrichtung wird z.B. in US 8257420 B2 beschrieben.

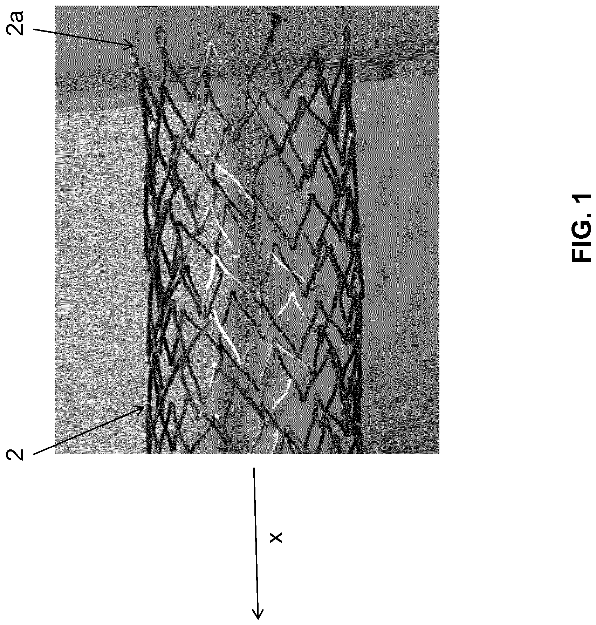

Bei derartigen Kathetersystemen, bei denen eine Kraft über ein Ende des Stents in den Stent eingeleitet wird, z.B. über einen Stentstopper, besteht insbesondere bei vergleichsweise langen Stents aus einer Vielzahl an miteinander verbundenen Segmenten das Problem, dass die Kraftübertragung von Segment zu Segment (ausgehend vom proximalen Stentende) zu einer unerwünschten Unordnung der einzelnen Segmente des Stents führen kann, die beispielhaft in der Figur 1 dargestellt ist. Die erzeugte Unordnung kann zur Störung der Freigabe des Stents, d.h., zu einer Überlappung einzelner Segmente, zur Blockierung des Stents oder sogar zu einer lediglich teilweisen Freisetzung (sogenannte "Partial Release") vom Stent im Körper führen.

Hiervon ausgehend liegt daher der Erfindung die Aufgabe zugrunde, ein Kathetersystem zu schaffen, das hinsichtlich der oben genannten Problematik verbessert ist.

Demgemäß wird ein Kathetersystem zum Implantieren eines selbstexpandierenden Stents vorgeschlagen, mit:
- einem selbstexpandierenden, in einer axialen Richtung erstreckten Stent, der dazu konfiguriert ist, sich durch Selbstexpansion aus einem komprimierten Zustand in einen expandierten Zustand zu expandieren,
- einem Katheter, der einen Außenschaft aufweist, der ein Lumen umgibt und eine dem Lumen zugewandte Innenseite aufweist, wobei der Stent im komprimierten Zustand in einem distalen Endabschnitt des Lumens anordenbar ist, so dass er an der Innenseite des Außenschaftes anliegt,
- einem im Lumen angeordneten Innenschaft des Katheters, wobei der Stent in dem komprimierten Zustand den Innenschaft umgibt, und wobei der Außenschaft bezüglich des Innenschaftes aus einer ersten Position in eine zweite Position bewegbar ist, wobei der Stent bei der Bewegung des Außenschaftes aus der ersten Position in die zweite Position freisetzbar ist und durch Selbstexpansion aus dem komprimierten Zustand in den expandierten Zustand expandiert wird, und
- einem am Innenschaft festgelegten Stentstopper, der dazu konfiguriert ist, zum Freisetzen des Stents bei der Bewegung des Außenschaftes aus der ersten Position in die zweite Position in der axialen Richtung gegen ein proximales Ende des Stents zu drücken.

Erfindungsgemäß ist nun zwischen dem Stent und dem Innenschaft des Katheters ein separates Abstützelement vorgesehen, das den Innenschaft umgibt und das dazu ausgebildet ist, den Stent beim Bewegen des Außenschaftes aus der ersten Position in die zweite Position abzustützen, wenn der Stentstopper in der axialen Richtung gegen das proximale Ende des Stents drückt.
Das erfindungsgemäße Design des Kathetersystems führt somit zu einer besseren Haltung des Stents während der Freigabe aus dem Katheter und reduziert das Risiko des sogenannten "Partial Release". Eine Überbelastung des Stents beim Freisetzen und die Deformation des freigesetzten Stents werden ausgeschlossen.

Die erfindungsgemäße Lösung verzichtet insbesondere mit Vorteil auf eine Erhöhung des Außendurchmessers des Innenschaftes, was sich positiv auf die Führbarkeit (sogenannte trackability) des Katheters auswirkt. D.h., der Katheter wird nicht zu steif und kann gut durch gekrümmte Gefäßabschnitte bewegt werden.

Gemäß einer Ausführungsform des erfindungsgemäßen Kathetersystems ist vorgesehen, dass das Abstützelement ein proximales Ende aufweist, das am Stentstopper anliegt.

Im Rahmen der vorliegenden Erfindung bedeutet distal, dass ein als distal bezeichneter Bereich oder eine als distal bezeichnete Komponente des Kathetersystems entlang des Katheters weiter vom Verwender, insbesondere Arzt, des Kathetersystems entfernt ist, als ein entsprechender als proximaler bezeichneter Bereich bzw. eine entsprechende als proximal bezeichnete Komponente.

Weiterhin ist gemäß einer Ausführungsform des erfindungsgemäßen Kathetersystems ist vorgesehen, dass das Abstützelement wendelförmig ausgebildet ist.

Das Abstützelement kann dabei ausgehend vom Stentstopper z.B. linksgängig oder rechtsgängig ausgebildet sein.

Weiterhin ist gemäß einer Ausführungsform der Erfindung vorgesehen, dass das Abstützelement einen Durchmesser im Bereich von 0,08mm bis 0,12mm, insbesondere 0,1 mm, aufweist.

Weiterhin ist gemäß einer Ausführungsform des erfindungsgemäßen Kathetersystems vorgesehen, dass das wendelförmige Abstützelement eine Ganghöhe im Bereich von 1 mm bis 2 mm aufweist.

Gemäß einer alternativen Ausführungsform des erfindungsgemäßen Kathetersystems ist vorgesehen, dass das schlauchförmig und nicht durchgängig ausgebildet ist und eine umlaufende Wandung aufweist. Nicht durchgängig ist so zu verstehen, dass die umlaufende Wandung Löcher oder Öffnungen umfasst und nicht als röhrenförmiger Schlauch mit vollständiger Wandung ausgebildet.

Gemäß einer Ausführungsform der Erfindung ist diesbezüglich vorgesehen, dass die Wandung eine Vielzahl an Durchgangsöffnungen aufweist.

Weiterhin ist gemäß einer Ausführungsform der Erfindung vorgesehen, dass die Wandung eine Netzstruktur aufweist oder durch eine Netzstruktur gebildet ist.

Weiterhin ist gemäß einer Ausführungsform der Erfindung vorgesehen, dass die Wandung des Abstützelements ein Geflecht aufweist oder durch ein Geflecht gebildet ist.

Ein Geflecht hat hierbei den besonderen Vorteil, dass dem Abstützelement ein sehr hohes Maß an Biegsamkeit und Flexibilität verliehen wird.

Weiterhin ist gemäß einer Ausführungsform der Erfindung vorgesehen, dass das Abstützelement oder die Wandung aus einem der folgenden Stoffe gefertigt ist oder eines der folgenden Stoffe aufweist: einen Kunststoff, ein Polymer, ein Polyamid, ein thermoplastisches Elastomer, thermoplastische Copolyamide, wie z.B. PEBAX. Durch die Verwendung von nachgiebigen Materialien wird gewährleistet, dass der Katheterteil, der den Stent und Abstützelement umfasst, eine geeignete Trackability aufweist.

Weiterhin ist gemäß einer Ausführungsform der Erfindung vorgesehen, dass der Stent dazu konfiguriert ist im peripheren Bereich eines Patienten implantiert zu werden, d.h. z.B. in einem Arm oder einem Bein des Patienten. So kann der Stent z.B. zur Behandlung atheriosklerotischer Erkrankungen der Arteria Femoralis oder der Arterien unterhalb der Arteria Poplitea ausgebildet sein. Weitere Indikationen wären Stent-Implantation in Venen, Speiseröhre, Luftröhre und den Gallenwegen.

Weiterhin ist gemäß einer Ausführungsform der Erfindung vorgesehen, dass der Stent in der axialen Richtung eine Länge im Bereich von 80 mm bis 250 mm, insbesondere im Bereich von 100 mm bis 200 mm, aufweist. Insbesondere bei sehr langen Stent für periphere Anwendungen kommt die Katheteranordung wie hierin vorgeschlagen, zu tragen, da insbesondere bei diesen sehr langen Stents Freisetzungsprobleme, wie Verschiebungen und Verspannungen einzelner oder mehrerer Stentstrutelemente, auftreten können.

Weiterhin ist gemäß einer Ausführungsform der Erfindung vorgesehen, dass der Stent eine Mehrzahl an Ringstrukturen aufweist, die jeweils in einer Umfangsrichtung des Stents verlaufen, wobei je zwei benachbarte Ringstrukturen über zumindest zwei Verbindungen, insbesondere drei Verbindungen, miteinander verbunden sind.

Weiterhin ist gemäß einer Ausführungsform der Erfindung vorgesehen, dass z.B. die in Umfangsrichtung verlaufenden bzw. umlaufenden Ringstrukturen sich jeweils in der axialen Richtung hin und her erstrecken bzw. in der Umfangsrichtung alternierend angeordnete Maxima und Minima aufweisen, wobei insbesondere die Maxima und Minima bezüglich der axialen Richtung vorliegen.

Weiterhin ist gemäß einer Ausführungsform der Erfindung vorgesehen, dass das Kathetersystem einen Führungsdraht aufweist.

Weiterhin ist diesbezüglich gemäß einer Ausführungsform der Erfindung vorgesehen, dass der Innenschaft ein Führungsdrahtlumen zur Aufnahme des Führungsdrahts bildet, so dass insbesondere der Katheter bzw. der Innenschaft entlang des Führungsdrahts zum Implantationsort des Stents vorgeschoben werden kann.

Im Folgenden sollen weitere Merkmale, Vorteile und Ausführungsformen der vorliegenden Erfindung anhand der Figuren erläutert werden. Es zeigen:
- Fig. 1: einen Stent, der durch Einleiten einer Kraft in das proximale Ende des Strents deformiert wird;
- Fig. 2: einen Stent eines erfindungsgemäßen Kathetersystems im expandierten Zustand;
- Fig. 3: den in der Fig. 2 gezeigten Stent im komprimierten (gecrimpten) Zustand;
- Fig. 4: eine schematische Schnittansicht einer Ausführungsform eines erfindungsgemäßen Kathetersystems mit einem Abstützelement zum Abstützen des Stents beim Freisetzen des Stents;
- Fig. 5: eine weitere Ausführungsform eines netzschlauchartigen Abstützelements, das anstelle des wendelförmigen Abstützelement gemäß Figur 4 verwendet werden kann;
- Fig. 6: eine weitere Ausführungsform eines Abstützelements, mit einer Wandung, die durch ein Geflecht gebildet ist, wobei dieses Abstützelement anstelle des wendelförmigen Abstützelement gemäß Figur 4 verwendet werden kann; und
- Fig. 7: eine Abwandlung des in der Fig. 6 gezeigten Abstützelements, wobei das Abstützelement zusätzlich ein rohrförmiges Wandungselement aufweist, das z.B. an einer Innenseite der geflochtenen Struktur anliegt.

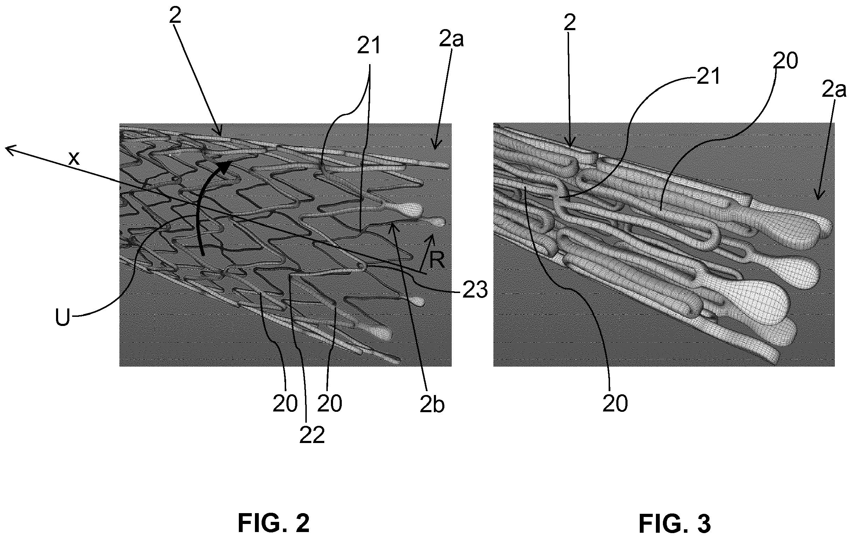

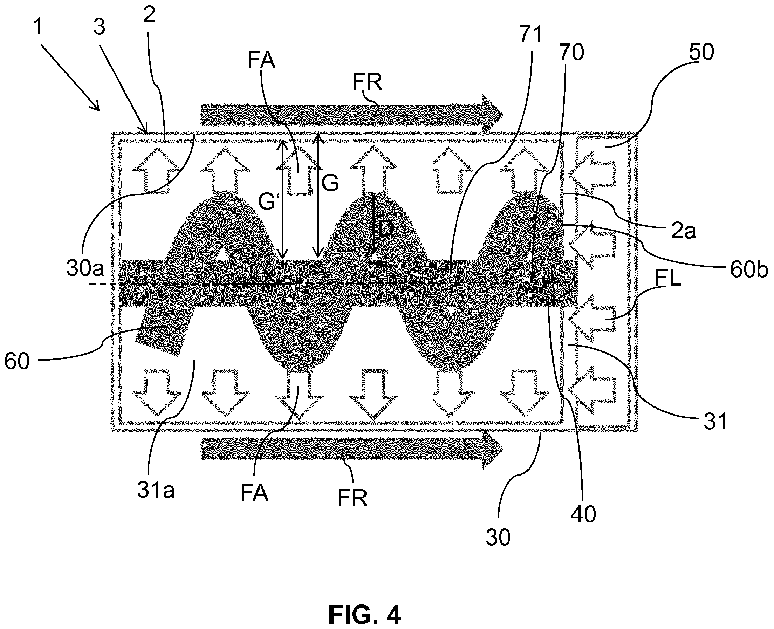

Fig. 4 zeigt eine Ausführungsform eines erfindungsgemäßen Kathetersystems 1 zum Implantieren eines selbstexpandierenden Stents 2 (vgl. auch Figuren 2 bis 3), wobei das Kathetersystem einen in einer axialen Richtung x erstreckten Stent 2 aufweist, der dazu konfiguriert ist, sich durch Selbstexpansion aus einem komprimierten Zustand in einen expandierten Zustand zu expandieren. Weiterhin weist das Kathetersystem 1 einen Katheter 3 auf, der einen Außenschaft 30 aufweist, der ein Lumen 31 umgibt und eine dem Lumen 31 zugewandte Innenseite 30a aufweist, wobei der Stent 2 im komprimierten bzw. gecrimpten Zustand in einem distalen Endabschnitt 31a des Lumens 31 angeordnet ist und insbesondere an der Innenseite 30a des Außenschaftes 30 anliegt. Weiterhin weist der Katheter 3 einen im Lumen 31 des Außenschaftes angeordneten Innenschaft 40 auf, wobei der Stent 2 gemäß Figur 4 in dem komprimierten Zustand (der z.B. in der Figur 3 gezeigt ist) den Innenschaft 40 umgibt. Zum Freisetzen des Stents 2 ist der Außenschaft 30 bezüglich des Innenschaftes 40 aus einer ersten Position in eine zweite Position bewegbar, so dass der Außenschaft den Stent 2 nicht mehr überdeckt bzw. zurückhält und der Stent 2 entsprechend durch Selbstexpansion aus dem komprimierten Zustand in den expandierten Zustand expandierbar ist, der in der Figur 2 gezeigt ist.

Zum Freisetzen des Stents ist ein am Innenschaft 40 festgelegter Stentstopper 50 vorgesehen, der dazu konfiguriert ist, bei der Bewegung des Außenschaftes 30 aus der ersten Position in die zweite Position in der axialen Richtung x gegen ein proximales Ende 2a des Stents 2 zu drücken, so dass der Stent bezüglich des Innenschaftes 40 in der axialen Richtung im Wesentliche seine Position behält, wenn der Außenschaft 30 in die zweite Position zurückgezogen wird. Der Stentstopper 50 übt also eine Lastkraft FL auf das proximale Ende 2a des Stents aus, die entgegen der von der Innenseite 30a des Außenschaft 30 auf den Stent ausgeübten Reibungskraft FR wirkt.

Derartige Kräfte können gemäß Figur 1 zu einer übermäßigen Deformation bzw. Unordnung der Stentstruktur 2 führen, was eine ordnungsgemäße Freisetzung des Stents 2 gefährden kann.

Erfindungsgemäß ist daher zwischen dem Stent 2 und dem Innenschaft 40 ein separates Abstützelement 60 vorgesehen, das den Innenschaft 40 umgibt und das dazu ausgebildet ist, den Stent 2 beim Bewegen des Außenschaftes 30 aus der ersten Position in die zweite Position abzustützen, wenn der Stentstopper 50 in der axialen Richtung x gegen das proximale Ende 2a des Stents 2 drückt. Hierzu kann das Abstützelement 60 in radialer Richtung R des Stents 2 eine Abstützkraft (auch als Nominalkraft bezeichnet) auf eine Innenseite 2b des Stents 2 ausüben (vgl. Fig 2), die der oben beschriebenen Deformation bzw. Unordnung des Stents 2 entgegenwirkt. Insbesondere wird hierdurch das Risiko gemindert, dass sich einzelne Segmente 20 bzw. Ringstrukturen 20 des Stents 2 in der axialen Richtung übereinander schieben.

Gemäß der in der Figur 4 gezeigten Ausführungsform kann das Abstützelement 60 wendelförmig ausgebildet sein, d.h., in bildet einen Wendel, wobei ein proximales Ende 60b des Wendels 60 vorzugweise bündig am Stentstopper 50 anliegt. Das Abstützelement kann z.B. einen Durchmesser D von 0,1mm aufweisen.

Das wendelförmige Abstützelement 60 kann z.B. aus einem Polyamid oder Pebax gefertigt sein.

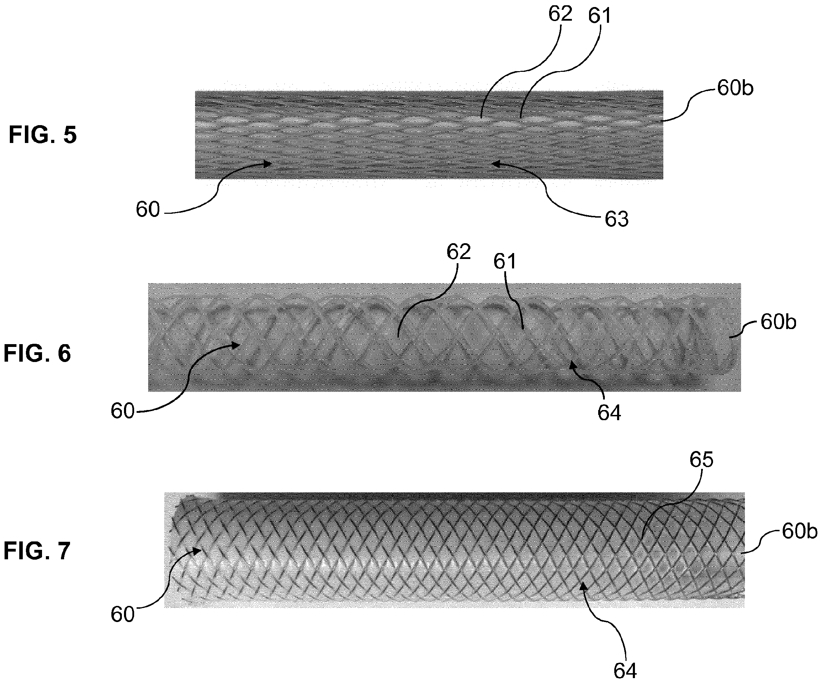

Die Figuren 5 bis 6 zeigen alternative Ausführungsformen des Abstützelements 60, die jeweils anstelle des wendelförmigen Abstützelements gemäß Figur 4 eingesetzt werden können.

Gemäß Figur 5 kann das Abstützelement 60 schlauchförmig ausgebildet sein und dabei eine umlaufende Wandung 61 aufweisen, die eine Vielzahl an Durchgangsöffnungen 62 ausbildet, wobei die Wandung 61 eine Netzstruktur 63 aufweist bzw. bildet. Das Abstützelement 60 kann also z.B. als Netzschutzschlauch ausgebildet sein. Insbesondere kann die Wandung 61 eine Wandstärke von z.B. 0,1 mm aufweisen. Das Abstützelement 60 kann über den Innenschaft 40 gezogen werden und dort z.B. mittels einer Klebeverbindung fixiert werden. Auch hier wird das proximale Ende 60b vorzugsweise wird bündig mit dem Stentstopper 50 positioniert. Die Länge des Abstützelements 60 in der axialen Richtung x kann der Länge des Stents 2 in dieser Richtung x entsprechen. Das Abstützelement 60 gemäß Figur 5 kann z.B. aus einem Kunststoff extrudiert sein (z.B. Polyamid, Pebax oder ähnliche Polymere).

Gemäß der in der Figur 6 gezeigten Ausführungsform kann das Abstützelement 60 auch eine Wandung 61 in Form eines Geflechts (oder auch in Form eines Gewebes) aufweisen. Im Falle eines Geflechts 64 wird das Abstützelement auch als "Braid" bezeichnet. Auch hier kann das Abstützelement 60 z.B. eine Wandstärke von 0,1 mm aufweisen und kann des Weiteren aus den oben bereits genannten Materialien gefertigt sein.

Weiterhin kann das Abstützelement 60 gemäß Figur 7 zusätzlich ein Wandelement 65 aufweisen, das das Geflecht 64 zumindest abschnittsweise an einer Innenseite oder an einer dem Stent 2 zugewandten Außenseite des Abstützelements 60 abdeckt. Solche Abstützelemente 60 werden auch als "Covered Braid" bezeichnet.
Im Rahmen der vorliegenden Erfindung handelt es sich bei dem Stent 2 insbesondere um einen Stent 2, der dazu konfiguriert ist, im peripheren Bereich eines Patienten implantiert zu werden (siehe auch oben).

Der Stent 2 kann in der axialen Richtung x eine vergleichsweise große Länge im Bereich von 80 mm bis 250 mm, insbesondere im Bereich von 100 mm bis 200 mm aufweisen.

Wie in den Figuren 2 und 3 gezeigt, kann der Stent 2 des Weiteren eine Mehrzahl an Ringstrukturen 20 aufweisen, die jeweils in einer Umfangsrichtung U des Stents 2 umlaufen, wobei je zwei benachbarte Ringstrukturen 20 über z.B. drei Verbindungen 21 miteinander verbunden sind. Hierbei können die in Umfangsrichtung U erstreckten Ringstrukturen 20 in der Umfangsrichtung U alternierend angeordnete Maxima 22 und Minima 23 aufweisen.

Aufgrund der erfindungsgemäßen Ausgestaltung des Kathetersystems 1 wird das Risiko eines Ordnungsverlusts des Stents 2 beim Freisetzen erheblich reduziert. Damit wird die Freigabe sicherer und das Risiko eines "Partial Release" kann vermieden werden.

Aufgrund der Abstützelemente 60 (z.B. Wendel, Netz oder Braid) bleibt der Katheter 3 insgesamt gut biegbar, so dass die Flexibilität des Katheters 3 nicht beeinträchtigt wird.

## Patentansprüche

1. Kathetersystem (1) zum Implantieren eines selbstexpandierenden Stents (2), mit:
- einem in einer axialen Richtung (x) erstreckten Stent (2), der dazu konfiguriert ist, sich durch Selbstexpansion aus einem komprimierten Zustand in einen expandierten Zustand zu expandieren,
- einem Katheter (3), der einen Außenschaft (30) aufweist, der ein Lumen (31) umgibt und eine dem Lumen (31) zugewandte Innenseite (30a) aufweist, wobei der Stent (2) im komprimierten Zustand in einem distalen Endabschnitt (31a) des Lumens (31) angeordnet ist und an der Innenseite (30a) des Außenschaftes (30) anliegt,
- einem im Lumen (31) angeordneten Innenschaft (40) des Katheters (3), wobei der Stent (2) in dem komprimierten Zustand den Innenschaft (40) umgibt, und wobei der Außenschaft (30) bezüglich des Innenschaftes (40) aus einer ersten Position in eine zweite Position bewegbar ist, wobei der Stent (2) bei der Bewegung des Außenschaftes (30) aus der ersten Position in die zweite Position freisetzbar ist und durch Selbstexpansion aus dem komprimierten Zustand in den expandierten Zustand expandierbar ist, und
- einem am Innenschaft (40) festgelegten Stentstopper (50), der dazu konfiguriert ist, zum Freisetzen des Stents (2) bei der Bewegung des Außenschaftes (30) aus der ersten Position in die zweite Position in der axialen Richtung (x) gegen ein proximales Ende (2a) des Stents (2) zu drücken,
**dadurch gekennzeichnet,**
**dass** zwischen dem Stent (2) und dem Innenschaft (40) ein separates Abstützelement (60) vorgesehen ist, das den Innenschaft (40) umgibt und das dazu ausgebildet ist, den Stent (2) beim Bewegen des Außenschaftes (30) aus der ersten Position in die zweite Position abzustützen, wenn der Stentstopper (50) in der axialen Richtung (x) gegen das proximale Ende (2a) des Stents (2) drückt.

2. Kathetersystem nach Anspruch 1, **dadurch gekennzeichnet, dass** das Abstützelement (60) ein proximales Ende (60b) aufweist, das am Stentstopper (50) anliegt.

3. Kathetersystem nach Anspruch 1 oder 2, **dadurch gekennzeichnet, dass** das Abstützelement (60) wendelförmig ausgebildet ist.

4. Kathetersystem nach einem der vorhergehenden Ansprüche, **dadurch gekennzeichnet, dass** das Abstützelement (60) einen Durchmesser (D) im Bereich von 0,08 mm bis 0,12 mm, insbesondere 0,1 mm, aufweist.

5. Kathetersystem nach einem der Ansprüche 3 bis 4, **dadurch gekennzeichnet, dass** das wendelförmige Abstützelement (60) eine Ganghöhe im Bereich von 1 mm bis 2 mm aufweist.

6. Kathetersystem nach Anspruch 1 oder 2, **dadurch gekennzeichnet, dass** das Abstützelement (60) schlauchförmig ausgebildet ist und eine umlaufende Wandung (61) aufweist.

7. Kathetersystem nach Anspruch 6, **dadurch gekennzeichnet, dass** die Wandung (61) eine Vielzahl an Durchgangsöffnungen (62) ausbildet.

8. Kathetersystem nach Anspruch 6 oder 7, **dadurch gekennzeichnet, dass** die Wandung (61) eine Netzstruktur (63) aufweist oder durch eine Netzstruktur (63) gebildet ist.

9. Kathetersystem nach Anspruch 6 oder 7, **dadurch gekennzeichnet, dass** die Wandung (61) ein Geflecht (64) aufweist oder durch ein Geflecht (64) gebildet ist.

10. Kathetersystem nach Anspruch 6 oder 7, **dadurch gekennzeichnet, dass** die Wandung (61) ein Gewebe aufweist oder durch ein Gewebe gebildet ist.

11. Kathetersystem nach einem der vorhergehenden Ansprüche, **dadurch gekennzeichnet, dass** das Abstützelement (60) aus einem der folgenden Stoffe gefertigt ist oder eines der folgenden Stoffe aufweist: einen Kunststoff, ein Polymer, ein Polyamid, ein thermoplastisches Elastomer, thermoplastische Copolyamide.

12. Kathetersystem nach einem der vorhergehenden Ansprüche, **dadurch gekennzeichnet, dass** der Stent (2) dazu konfiguriert ist, im peripheren Bereich eines Patienten implantiert zu werden.

13. Kathetersystem nach einem der vorhergehenden Ansprüche, **dadurch gekennzeichnet, dass** der Stent (2) in der axialen Richtung (x) eine Länge im Bereich 80 mm bis 250 mm, insbesondere im Bereich von 100 mm bis 200 mm aufweist.

14. Kathetersystem nach Anspruch 1 oder 2, **dadurch gekennzeichnet, dass** der Stent (2) eine Mehrzahl an Ringstrukturen (20) aufweist, die jeweils in einer Umfangsrichtung (U) des Stents (2) verlaufen, wobei je zwei benachbarte Ringstrukturen (20) über zumindest zwei Verbindungen (21) miteinander verbunden sind.

15. Kathetersystem nach Anspruch 1 oder 2, **dadurch gekennzeichnet, dass** die in Umfangsrichtung verlaufenden Ringstrukturen (20) in der Umfangsrichtung (U) alternierend angeordnete Maxima (22) und Minima (23) aufweisen.
